# EUROPÄISCHE PATENTSCHRIFT

(11) **EP 3 329 902 B1**
(45) Veröffentlichungstag und Bekanntmachung des Hinweises auf die Patenterteilung: **19.02.2020**
(21) Anmeldenummer: 17202704.7
(22) Anmeldetag: 21.11.2017
(51) Int. Cl.: A61K 8/37, A61K 8/46, A61K 8/60, A61Q 19/10, A61K 8/04

(54) **SCHÄUMENDES REINIGUNGSPRODUKT AUF EMULSIONSBASIS**
FOAMING EMULSION-BASED CLEANSING PRODUCT
PRODUIT NETTOYANT MOUSSANT À BASE D'ÉMULSION

(30) Priorität: 02.12.2016 DE 102016224029
(43) Veröffentlichungstag der Anmeldung: 06.06.2018
(73) Patentinhaber: Beiersdorf AG, 20253 Hamburg (DE)
(72) Erfinder: Johns, Nicole, 22958 Kuddewörde (DE); Max, Heiner, 22529 Hamburg (DE); Möllgaard, Svenja Lena, 22337 Hamburg (DE); Schäfer, Jessica, 22113 Oststeinbek (DE)

(56) Entgegenhaltungen:
- WO-A1-2016/170246
- DE-A1-102014 217 530
- FR-A1- 3 016 884
- US-B1- 9 333 153

## Beschreibung

Die vorliegende Erfindung betrifft ein kosmetisches Reinigungsprodukt aufweisend einen Druckbehälter mit Entnahmeventil enthaltend ein Treibgas oder Treibgasgemisch und eine Emulsion, umfassend ein Aminosäuretensid und/oder ein Alkylsulfat, Glycerylmonostearat SE und mindestens ein Alkylpolyglycosid. Bei Entnahme der mit Treibgas ausgetragenen und aufgeschäumten erfindungsgemäßen Emulsion wird ein strukturell besonders stabiler Schaum enthalten.

Schön und attraktiv auszusehen ist ein Bedürfnis von vielen Menschen. Oftmals gilt dabei eine reine und gepflegte Haut als Schönheitsideal. Um diesem zu entsprechen, werden die verschiedensten kosmetischen Produkte zur täglichen Reinigung und Pflege der Haut eingesetzt.

Die Reinigung der Haut dient dazu Schmutz, Schweiß und Reste abgestorbener Körperzellen, die als Nährstoffe für Krankheitserreger und Parasiten aller Art dienen können, zu entfernen. Dazu werden oftmals kosmetische Zubereitungen, die als Emulsion formuliert sind, eingesetzt.

Unter Emulsionen versteht man im Allgemeinen heterogene Systeme, die aus zwei nicht oder nur begrenzt mischbaren Flüssigkeiten bestehen, wobei eine der beiden Flüssigkeiten in Form feinster Tröpfchen in der anderen Flüssigkeit dispergiert ist. Mit dem bloßen Auge erscheint eine Emulsion als homogen. Sind beide Flüssigkeiten Wasser und Öl, und liegt das Öl als fein verteilte Tröpfchen im Wasser vor, so handelt es sich um eine Öl-in-Wasser-Emulsion (O/W-Emulsion). Liegt hingegen das Wasser als fein verteilte Tröpfchen im Öl vor, so handelt es sich um eine Wasser-in-ÖI-Emulsion (W/O-Emulsion).

Wird nachfolgend der Begriff Haut verwendet so bezieht sich dieser ausschließlich auf die menschliche Haut.

Beim Waschvorgang wird die kosmetische Zubereitung zunächst auf die Haut appliziert und dort mit den Händen oder einem Hilfsmittel, wie einem Pad, Tuch oder ähnlichem, verrieben. Anschließend wird die kosmetische Zubereitung zusammen mit den gelösten Schmutz, Schweiß oder Resten abgestorbener Körperzellen entweder mit einem Hilfsmittel abgenommen oder mit Wasser abgespült.

Dabei sind beim Anwender insbesondere kosmetische Zubereitungen beliebt, die sich vor oder bei Anwendung auf der Haut aufschäumen lassen und eine große Menge Schaum produzieren. Alternativ zu den klassischen Reinigungsprodukten, die zur Reinigung der Haut im flüssigen Zustand aus dem Vorratsbehältnis entnommen und auf der Haut aufgetragen werden, gibt es eine geringe Anzahl an Produkten, bei denen die Zubereitung zur Anwendung auf der Haut mit Hilfe von Treibgas aufgeschäumt wird. Dabei wird ein Schaum mit moussiger Textur erhalten, der dem Anwender nicht nur Spaß bei Auftragen auf die Haut bereitet, sondern auch zumeist als gut auf der Haut verteilbar wahrgenommen wird.

Nachteilig am Stand der Technik ist jedoch der Umstand, dass mit Treibgas aufgeschäumte kosmetische Reinigungszubereitungen oftmals eine unzureichende strukturelle Stabilität aufweisen und so nach dem ausbringen schnell in sich zusammenfallen, beziehungsweise sich auflösen.

Wird in der vorliegenden Offenbarung von einer guten beziehungsweise schlechten strukturellen Stabilität des Schaumes gesprochen, so ist dies so zu verstehen, dass der Schaum besonders langsam beziehungsweise besonders schnell in sich Zusammenfällt und sich langsam beziehungsweise schnell aufzulösen beginnt.

Nachteilig ist ferner, dass sich insbesondere Reinigungsemulsionen oftmals nur schlecht mit Treibgas aufschäumen lassen und so ein unzureichender Schaum erhalten wird, welcher grobblasig und/oder dünnflüssig ist und/oder schnell zusammenfällt.

Ein weiterer Nachteil an vielen Reinigungsschäumen ist, dass der erhaltenen Schaum nur eine geringe Tragkraft hat und sich so bei der Anwendung sehr luftig anfühlt. Wünschenswert sind jedoch Schäume, welche eine höhere Tragkraft aufweisen und so bei Anwendung auf der Haut ein cremigeres Gefühl hervorrufen.

Überraschend wurde nun gefunden, dass die Nachteile des Standes der Technik durch den Gegenstand der vorliegenden Erfindung gelöst werden konnten.

Die Erfindung ist ein kosmetisches Reinigungsprodukt aufweisend einen Druckbehälter mit Entnahmeventil enthaltend
a) eine Emulsion umfassend
   i) mindestens ein Aminosäuretensid gemäß einer der Formeln I bis IV 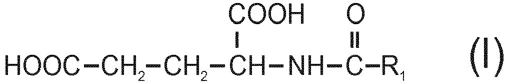 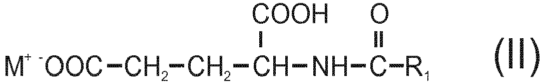 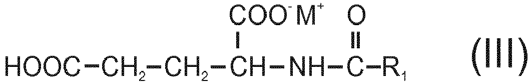 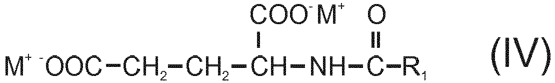 wobei *R₁* eine Alkylkette mit 10 bis 20 Kohlenstoffatomen ist und *M*⁺ jeweils ein Na⁺ oder NH₄⁺ ist,
      und/oder mindestens ein Alkylsulfat gemäß der Formel V 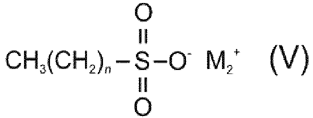 wobei n eine ganzzahlige Zahl im Bereich von 9 bis 19 ist und *M*₂⁺ ein Na⁺ oder NH₄⁺ ist,
   ii) Glycerylmonostearat SE, und
   iii) mindestens ein Alkylpolyglycosid
   und
b) mindestens ein Treibgas oder Treibgasgemisch.

Auch Gegenstand der Erfindung ist die Verwendung einer mit einem Treibgas oder Treibgasgemisch aufgeschäumten Emulsion umfassend
i) mindestens ein Aminosäuretensid gemäß einer der Formeln I bis IV 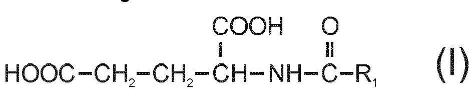 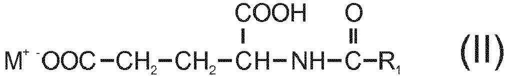 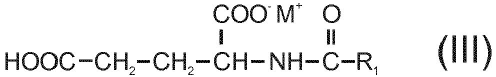 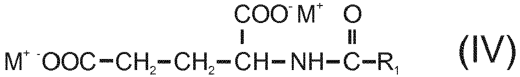 wobei *R₁* eine Alkylkette mit 10 bis 20 Kohlenstoffatomen ist und *M*⁺ jeweils ein Na⁺ oder NH₄⁺ ist,
   und/oder mindestens ein Alkylsulfat gemäß der Formel V 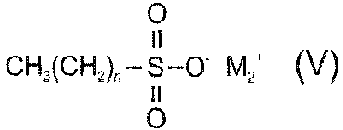 wobei n eine ganzzahlige Zahl im Bereich von 9 bis 19 ist und *M*₂⁺ ein Na⁺ oder NH₄⁺ ist,
ii) Glycerylmonostearat SE, und
iii) mindestens ein Alkylpolyglycosid
zur Bereitstellung eines Schaumes mit einer verbesserten Tragkraft und/oder höheren strukturellen Stabilität.

In EP 1277455 A1 werden verschiedene aufgeschäumte O/W-Emulsionen offenbart. Keines der Beispiele enthält die erfindungsgemäße Kombination an Tensiden und Emulgatoren. Demensprechend ist dieser Schrift kein Hinweis auf die vorliegende Erfindung zu entnehmen. Im weiteren Stand der Technik wird eine Vielzahl an Emulgatoren in Emulsionen eingesetzt. So sind aus DE 19645319 A1 schäumende Zubereitungen mit Polyglyceryl-3 Methylglucose Distearat als Emulgator bekannt. Es wurde nun gefunden, dass sich Emulsionen enthaltend diesen Emulgator schlecht mit Treibgas aufschäumen lassen, der erhaltene Schäum schnell zerfällt und damit keine strukturelle Stabilität zeigt. Ein Einsatz der erfindungsgemäßen Emulsion ergibt hingegen überraschend Schäume mit einer deutlich verbesserten strukturellen Stabilität.

Des Weiteren kennt der Stand der Technik die Dokumente WO 9908649 A2 und DE10138495 A1, doch konnten auch diese Schriften nicht den Weg zur vorliegenden Erfindung weisen. Desweiteren offenbart das Dokument WO 2016/170246 A1 kosmetische Schaumzubereitungen.

Das erfindungsgemäße kosmetische Reinigungsprodukt bildet nach ausbringen einen überraschend feinblasigen, gleichmäßigen, strukturell stabilen und cremigen Schaum.

Alle nachfolgend aufgeführten Gewichtsprozentangaben (Gew.-%) beziehen sich, sofern nicht anders angegeben, jeweils auf das Gesamtgewicht der Emulsion.

Der Ausdruck "frei von" bedeutet im Sinne der vorliegenden Offenbarung, dass der Anteil der jeweiligen Substanz kleiner 0,05 Gew.-% ist. Dadurch wird gewährleistet, dass Einschleppungen oder Verunreinigungen mit diesen Stoffen nicht als erfindungsgemäß "frei von" mitumfasst werden.

Es ist erfindungsgemäß von Vorteil, wenn die erfindungsgemäße Emulsion mindestens ein Aminosäuretensid gemäß einer der Formeln I bis IV und/oder mindestens ein Alkylsulfat gemäß der Formel V in einem Gesamtanteil 0,05 bis 2 Gew.-%, bevorzugt 0,075 bis 0,5 Gew.-% bezogen auf das Gesamtgewicht der Emulsion enthält.

Erfindungsgemäß vorteilhaft werden als Aminosäuretenside gemäß der Formeln I bis IV Natriumlauroylglutamat, Dinatriumlauroylglutamat, Natriumcocoylglutamat, Dinatriumcocoylglutamat, Natriummyristoylglutamat, Dinatriummyristoylglutamat, Natriumcetoylglutamat, Dinatriumcetoylglutamat, Natriumstearoylglutamat und/oder Dinatriumstearoylglutamat gewählt. Ein insbesondere vorteilhafter Schaum hat sich bei Einsatz von Natriumstearoylglutamat gebildet. Demensprechend ist es insbesondere bevorzugt, wenn als Aminosäuretensid Natriumstearoylglutamat enthalten ist.

Es ist ebenfalls erfindungsgemäß vorteilhaft, wenn als Alkylsulfat Natriumlaurylsulfat, Ammoniumlaurylsulfat, Natriummyristylsulfat, Natriumcetylsulfat, Natriumcetearylsulfat und/oder Natriumstearylsulfat gewählt werden. Als insbesondere bevorzugt hat sich Natriumcetearylsulfat erwiesen.

Insbesondere vorteilhafte Ausführungsformen der Erfindung enthalten somit Natriumstearoylglutamat und Natriumcetearylsulfat.

Eine weitere insbesondere vorteilhafte Ausführungsform der Erfindung enthält ausschließlich Natriumcetearylsulfat und der Anteil aller weiteren unter i) fallenden Substanzen ist kleiner 0,01 Gew.-%. In dieser Ausführungsform hat es sich überraschend gezeigt, dass nicht nur ein überaus strukturell stabiler Schaum erhalten wird, sondern auch, dass das Korrosionspotential mit Druckbehältern, die Aluminiumbestandteile aufweisen, deutlich reduziert wurde.

Es ist erfindungsgemäß vorteilhaft, wenn die erfindungsgemäße Emulsion Glycerylmonostearat SE in einem Anteil von 0,1 bis 5 Gew.-%, bevorzugt 1 bis 4 Gew.-% und insbesondere bevorzugt 1,5 bis 3 Gew.-%, bezogen auf das Gesamtgewicht der Emulsion, enthält.

Erfindungsgemäß wird selbstemulgierendes Glycerylmonostearat SE mit der INCI Bezeichnung Glyceryl Stearate SE eingesetzt. Dieses ist unter anderem unter dem Handelsnamen TEGIN® Pellets von Evonik Industries AG oder Cutina® GMS-SE von der BASF zu beziehen.

Es ist vorteilhaft im Sinne der vorliegenden Erfindung, wenn das Gewichtsverhältnis in der Emulsion von den mindestens einen Aminosäuretensid gemäß einer der Formeln I bis IV und/oder mindestens ein Alkylsulfat gemäß der Formel V zu dem Gewichtsanteil von Glycerylmonostearat SE 1:1 bis 1:20, vorteilhaft 1:10 bis 1:18 beträgt.

Ferner ist es insbesondere vorteilhaft, wenn der Gesamtanteil der Alkylpolyglycoside in der erfindungsgemäßen Emulsion von 0,05 Gew-% bis 5 Gew.-%, bevorzugt 0,1 Gew-% bis 2 Gew.-% und insbesondere bevorzugt 0,2 Gew-% bis 1 Gew.-% bezogen auf das Gesamtgewicht der erfindungsgemäßen Emulsion beträgt.

In die Stoffgruppe der Alkylpolyglycoside fallen erfindungsgemäß alle Substanzen gemäß der Formel VI 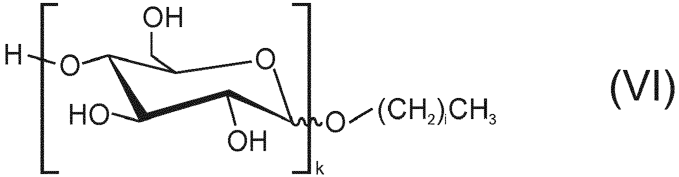 wobei *k* eine ganzzahlige Zahle von 1 bis 5 ist und in der Alkylkette *i* eine ganzzahle Zahl von 5 bis 20 ist. Erfindungsgemäße Beispiele diese Alkylpolyglycoside sind unter dem Handelsnamen Plantacare 818 UP, Plantacare 2000 UP und Plantacare 1200 UP von der BASF zu beziehen. Plantacare 818 UP weist die INCI-Bezeichnung Coco Glucoside auf. Plantacare 2000 UP ist unter der INCI Bezeichnung Decyl Glucoside bekannt. Plantacare 1200 P wird als Lauryl Glucoside bezeichnet.

Besonders vorteilhafte werden Mischungen von Alkylpolyglycoside eingesetzt, die dadurch gekennzeichnet sind, dass die in der Emulsion enthaltenen Alkylpolyglycoside die nachfolgenden Anteile an Alkylketten aufweisen:
i=7 in einem Anteil von 24 Gew.-% bis 40 Gew.-%
i=9 in einem Anteil von 15 Gew.-% bis 28 Gew.-%
i=11 in einem Anteil von 27 Gew.-% bis 42 Gew.-%
i=13 in einem Anteil von 9 Gew.-% bis 18 Gew.-%
i=15 in einem Anteil von maximal 4 Gew.-%
wobei sich die vorstehenden Gew.-% Angaben auf das Gesamtgewicht aller in der Emulsion enthaltenen Alkylpolyglycoside beziehen.

Die erfindungsgemäße Emulsion ist ferner vorteilhaft dadurch gekennzeichnet, dass es sich um eine O/W-Emulsion handelt. O/W-Emulsionen enthaltend die erfindungsgemäße Kombination aus den Komponenten i) bis iii) tendieren überraschend weniger zu agglomerieren. Des Weiteren zeigt der erhaltene Schaum eine überraschend verbesserte strukturelle Stabilität.

Als Treibgas oder Treibgasgemisch werden erfindungsgemäß vorteilhaft die Treibgase der Gruppe Propan, n-Butan, Iso-Butan, Pentan und/oder Stickstoff eingesetzt. Weniger geeignet zu verwenden sind Fluorchlorkohlenwasserstoffe, welche die Umwelt beeinträchtigen und daher für die Anwendung in kosmetischen Produkten für den Massenmarkt nicht geeignet sind. Wird Pentan eingesetzt, so wird im Vergleich zu Propan, n-Butan und Iso-Butan ein sensorisch schlechterer Schaum erhalten. Demensprechend enthält das kosmetische Reinigungsprodukt vorteilhaft weder Pentan noch Fluorchlorkohlenwasserstoffe und bevorzugt sind ausschließlich Treibgase der Gruppe Propan, n-Butan und Iso-Butan enthalten.

Vorteilhaft werden die eingesetzten Treibgase je nach Druckstufe im kosmetischen Reinigungsprodukt in den folgenden Mischungsverhältnissen eingesetzt:
Druckstufe 2,7 bar: 60 Gew.-% n-Butan, 20 Gew.-% Propan und 20 Gew.-% Iso-Butan;
Druckstufe 3,0 bar: 5,3 Gew.-% n-Butan, 15,3 Gew.-% Propan und 79,4 Gew.-% Iso-Butan;
Druckstufe 3,5 bar: 5 Gew.-% n-Butan, 23 Gew.-% Propan und 72 Gew.-% Iso-Butan;
jeweils bezogen auf das Gesamtgewicht des gesamten Treibgasgemisches.

Erfindungsgemäß bevorzugt sind die Druckstufen 2,7 bar, 3,0 bar und 3,5 bar.

Besonders bevorzugt sind die Druckstufen 3,0 bar und 3,5 bar.

Unter dem Begriff "Druckstufe" wird der Druck bezeichnet unter dem bei 20°C die erfindungsgemäße Emulsion mit dem entsprechenden Treibgas oder Treibgasgemisch in dem Druckbehälter gelagert bzw. abgefüllt wird.

Es ist erfindungsgemäß von Vorteil, wenn im Druckbehälter 90 Gew.-% bis 98 Gew.-% Emulsion und 2 Gew.-% bis 10 Gew.-% Treibgas oder Treibgasgemisch, bezogen auf das Gesamtgewicht aller im Druckbehälter enthaltenen Flüssigkeiten und Gase, vorliegen. Dabei ist es erfindungsgemäß bevorzugt, wenn der Anteil der erfindungsgemäßen Emulsion 92 Gew.-% bis 96 Gew.-% und der Anteil des Treibgases oder des Treibgasgemisches 4 Gew.-% bis 8 Gew.-%, bezogen auf das Gesamtgewicht aller im Druckbehälter enthaltenen Flüssigkeiten und Gase, beträgt.

Ferner ist es erfindungsgemäß von Vorteil, wenn die erfindungsgemäße Emulsion ein oder mehr Veresterungsprodukte aus Glycerin mit drei identischen linearen und/oder verzweigten Fettsäuren mit einer Alkylkette mit 12 bis 20 Kohlenstoffatomen in einem Anteil von 0,01 Gew.-% bis 15 Gew.-%, bevorzugt 0,5 Gew.-% bis 10 Gew.-% und insbesondere bevorzugt 1 Gew.-% bis 5 Gew.-%, bezogen auf das Gesamtgewicht der Emulsion, enthält.

Erfindungsgemäß bevorzugte Veresterungsprodukte aus Glycerin mit drei identischen linearen und/oder verzweigten Fettsäuren Alkylkette mit 12 bis 20 Kohlenstoffatomen sind unter den INCI-Namen Tripalmitin, Tristearin und Triisostearin. Insbesondere bevorzugt ist Triisostearin.

Eine besonders bevorzugte Ausführungsform der vorliegenden Erfindung ist dadurch gekennzeichnet, dass die Emulsion Triisostearin in einem Anteil von 0,01 Gew.-% bis 15 Gew.-%, bevorzugt 0,5 Gew.-% bis 10 Gew.-% und insbesondere bevorzugt 1 Gew.-% bis 5 Gew.-% enthält und die Emulsion frei von weiteren Veresterungsprodukten von Glycerin mit drei identischen linearen und/oder verzweigten Fettsäuren mit einer Alkylkette mit 12 bis 20 Kohlenstoffatomen ist.

Die erfindungsgemäßen Veresterungsprodukte aus Glycerin mit drei identischen linearen und/oder verzweigten Fettsäuren mit einer Alkylkette mit 12 bis 20 Kohlenstoffatomen enthalten keine weiteren Alkoxy-Einheiten, wie beispielsweise Ethylenoxid oder Propylenoxid Gruppen.

Ferner ist es vorteilhaft im Sinne der vorliegenden Erfindung, wenn die erfindungsgemäße Emulsion keine weiteren Tenside enthält.

Ferner war es überraschend, dass die vorliegende Emulsion auch bei einem sehr geringen Tensidanteil von vorteilhaft weniger als 4 Gew.-%, bevorzugt weniger als 3,5 Gew.-% bezogen auf das Gesamtgewicht der Emulsion, bei Anwendung des Schaumes des kosmetischen Reinigungsproduktes auf der Haut eine besonders gute Reinigungsleistung erzielt. Zu dem Tensidanteil werden erfindungsgemäß auch die Substanzen i) und iii) der Emulsion gerechnet.

Erfindungsgemäß vorteilhafte Ausführungsformen der Erfindung sind ferner dadurch gekennzeichnet, dass die erfindungsgemäße Emulsion Phenoxyethanol, Benzethoniumchlorid und/oder Ethylhexylglycerin enthält.

Enthält die Emulsion Phenoxyethanol, so ist es erfindungsgemäß von Vorteil, wenn der Anteil an Phenoxyethanol 0,1 Gew.-% bis 1 Gew.-% bezogen auf das Gesamtgewicht der erfindungsgemäßen Emulsion beträgt.

Enthält die Emulsion Benzethoniumchlorid, so ist es erfindungsgemäß von Vorteil, wenn der Anteil an Benzethoniumchlorid 0,01 Gew.-% bis 0,4 Gew.-% bezogen auf das Gesamtgewicht der erfindungsgemäßen Emulsion beträgt.

Enthält die Emulsion Ethylhexylglycerin, so ist es erfindungsgemäß von Vorteil, wenn der Anteil an Ethylhexylglycerin 0,1 Gew.-% bis 2 Gew.-% bezogen auf das Gesamtgewicht der erfindungsgemäßen Emulsion beträgt.

Es ist erfindungsgemäß vorteilhaft, wenn die erfindungsgemäße Emulsion dadurch gekennzeichnet ist, dass die erfindungsgemäße Emulsion Benzylalkohol, Methylparaben und/oder Ethylparaben enthält.

Enthält die erfindungsgemäße Emulsion Benzylalkohol, so ist es erfindungsgemäß bevorzugt, diesen Stoff in einem Anteil von 0,1 Gew.-% bis 2 Gew.-% bezogen auf das Gesamtgewicht der erfindungsgemäßen Emulsion einzusetzen.

Enthält die erfindungsgemäße Emulsion Methylparaben, so ist es erfindungsgemäß bevorzugt, diesen Stoff in einem Anteil von 0,1 Gew.-% bis 1 Gew.-% bezogen auf das Gesamtgewicht der erfindungsgemäßen Emulsion einzusetzen.

Enthält die erfindungsgemäße Emulsion Ethylparaben, so ist es erfindungsgemäß bevorzugt, diesen Stoff in einem Anteil von 0,1 Gew.-% bis 1 Gew.-% bezogen auf das Gesamtgewicht der erfindungsgemäßen Emulsion einzusetzen.

Erfindungsgemäß vorteilhafte Ausführungsformen der vorliegenden Erfindung sind dadurch gekennzeichnet, dass die Fettphase der Emulsion ein oder mehrere Fettalkohole enthält, wobei Stearylalkohol, Cetylalkohol und/oder deren Mischungen bevorzugt gewählt werden.

Der erfindungsgemäß vorteilhafte Anteil von Stearylalkohol, Cetylalkohol und/oder deren Mischungen in der erfindungsgemäßen Emulsion beträgt 0,1 Gew.-% bis 2 Gew.-%, bezogen auf das Gesamtgewicht der erfindungsgemäßen Emulsion.

Des Weiteren sind erfindungsgemäß vorteilhafte Ausführungsformen der vorliegenden Erfindung dadurch gekennzeichnet, dass die Fettphase der Emulsion ein oder mehrere Ester von linearen oder verzweigten, gesättigten oder ungesättigten Alkoholen mit 2 bis 6 Kohlenstoffatomen mit linearen oder verzweigten, gesättigten oder ungesättigten Fettsäuren mit 12 bis 18 Kohlenstoffatomen enthält. Diese Ester werden bevorzugt gewählt aus der Gruppe Isopropylmyristat, Isopropylpalmitat, Isopropylstearat und/oder Isopropylisostearat.

Der erfindungsgemäß vorteilhafte Anteil von Isopropylmyristat, Isopropylpalmitat, Isopropylstearat und/oder Isopropylisostearat beträgt 2 Gew.-% bis 8 Gew.-%, bezogen auf das Gesamtgewicht der erfindungsgemäßen Emulsion.

Ferner sind erfindungsgemäß vorteilhafte Ausführungsformen der vorliegenden Erfindung dadurch gekennzeichnet, dass Dicaprylylether in einem Anteil von 1,5 Gew.-% bis 5 Gew.-%, bezogen auf das Gesamtgewicht der erfindungsgemäßen Emulsion, in der erfindungsgemäßen Emulsion enthalten ist.

Es ist erfindungsgemäß von Vorteil, wenn der Anteil der Fettphase der Emulsion 7,5 Gew.-% bis 15 Gew.-%, bezogen auf das Gesamtgewicht der erfindungsgemäßen Emulsion, beträgt.

Vorteilhaft kann die erfindungsgemäße Emulsion Glycerin enthalten. Es ist dabei erfindungsgemäß bevorzugt, wenn Glycerin in einem Anteil von 5 Gew.-% bis 15 Gew.-%, bezogen auf das Gesamtgewicht der erfindungsgemäßen Emulsion, enthalten ist.

Des Weiteren ist es vorteilhaft, wenn die erfindungsgemäße Emulsion Methylpropanediol enthält. Besonders vorteilhaft ist dabei Methylpropanediol in einem Anteil von 0,5 Gew.-% bis 4 Gew.-%, bezogen auf das Gesamtgewicht der erfindungsgemäßen Emulsion, enthalten.

Es ist erfindungsgemäß vorteilhaft, wenn die erfindungsgemäße O/W-Emulsion einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivat, Glycyrrhiza Inflata Root Extract, Magnolienextrakt enthält.

Erfindungsgemäß vorteilhafte Ausführungsformen der vorliegenden Erfindung sind dadurch gekennzeichnet, dass die erfindungsgemäße Emulsion frei von weiteren Seifen ist.

Erfindungsgemäß werden Seifen als Natrium- oder Kaliumsalze von Fettsäuren verstanden.

Da Seifen oftmals zur strukturellen Stabilisierung von Schäumen, wie beispielsweise bei Rasierschaum, eingesetzt werden, war es überraschend, dass das erfindungsgemäße kosmetische Reinigungsprodukt der vorteilhaften Ausführungsform strukturell stabile Schäume bildet. Seifenfreie kosmetische Reinigungsprodukte haben ferner den Vorteil, dass diese ein signifikant reduziertes Hautreizungspotential aufweisen.

Die erfindungsgemäße Emulsion hat vorteilhaft einen pH-Wert im Bereich von 5 bis 7.

Ferner ist die erfindungsgemäße Emulsion vorteilhaft dadurch gekennzeichnet, dass der Wachse und Öle in einem Anteil von weniger als 10 Gew.-%, bezogen auf das Gesamtgewicht der Emulsion enthalten sind. Höhere Öl- und Wachsanteile führen zu einem unvorteilhaften Schaum.

Des Weiteren ist es vorteilhaft, wenn die erfindungsgemäße Emulsion frei von UV-Filtern ist.

Im Sinne der vorliegenden Erfindung werden als UV-Filter die Substanzen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid bezeichnet.

Ferner ist es vorteilhaft, wenn die erfindungsgemäße Emulsion frei von Verdickungsmittel ist. Als Verdickungsmittel werden im Sinne der vorliegenden Erfindung Carbomere, Polysaccharide und Crosspolymere bezeichnet. Der Einsatz von Verdickungsmitteln würde zu Schäumen mit unvorteilhaften sensorischen Eigenschaften führen.

Erfindungsgemäß vorteilhafte Ausführungsformen der vorliegenden Erfindung sind dadurch gekennzeichnet, dass die erfindungsgemäße Emulsion frei von Mineralöl und/oder Silikonöl ist. Sowohl Mineralöle als auch Silikonöle können die Sensorik einer aufgeschäumten Emulsion beeinträchtigen.

Zur Anwendung wird das erfindungsgemäße kosmetische Reinigungsprodukt durch Schütteln des Druckbehälters zuerst durchmischt und anschließend über das Entnahmeventil entnommen und auf die Haut appliziert. Bei Entnahme aus dem Druckbehälter wird ein Schaum bzw. eine aufgeschäumte kosmetische Zubereitung erhalten.

Als Druckbehälter mit Entnahmeventil können die üblichen für kosmetische Zwecke bekannten Aerosoldosen-Systeme eingesetzt werden. Vorteilhaft weist der Druckbehälter mit Entnahmeventil einen geeigneter Sprüh bzw. Schaumkopf welcher das Austragen von schäumen erlaubt. Diese sind kommerziell erhältlich (zum Beispiel der APTAR Accessories Foam Upright S25 Foam). Das Material des Druckbehälters enthält vorzugsweise Aluminium.

Erfindungsgemäß ist daher auch ein Verfahren zur Anwendung des erfindungsgemäßen kosmetischen Reinigungsprodukts auf der Haut, welches dadurch gekennzeichnet ist, dass die Inhaltsstoffe des Druckbehälters durch Schütteln in des Druckbehälters zuerst durchmischt und anschließend über das Entnahmeventil entnommen und auf die Haut appliziert wird.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Vergleichsversuch:

Zum Vergleich wurden ein Schaum basierend auf dem im Stand der Technik (bspw. DE 19645319 A1) bekannten Emulgator Polyglyceryl-3 Methylglucose Distearat und mit dem Schaum einer erfindungsgemäßen Zubereitung enthaltend die Kombination der Substanzen i) bis iii) verglichen.

| **INCI** | **Erfindungsgemäß** | **Stand der Technik** |
|---|---|---|
| | **Erf. 1** | **SdT. 1** |
| **O/W-Emulsion:** | | |
| Panthenol (75% in Wasser) | 1,4 | 1,4 |
| Dicaprylyl Ether | 3 | 3 |
| Isopropyl Palmitate | 5 | 5 |
| Triisostearin | 2 | 2 |
| Glyceryl Stearate SE | 2,4 | - |
| Sodium Cetearyl Sulfate | 0,15 | - |
| Polyglyceryl-3 Methylglucose Distearate | - | 0,5 |
| Lanolin Alcohol | 0,1 | 0,1 |
| Octyldodecanol | 0,1 | 0,1 |
| Parfum | 0,25 | 0,25 |
| Glycerin (99% in Wasser) | 9,3 | 6 |
| Methylpropanediol | 2 | 2 |
| Citric Acid | 0,03 | - |
| Sodium Hydroxid (45% in Wasser) | - | 0,002 |
| Phenoxyethanol | 0,9 | 0,6 |
| Ethylhexylglycerin | 0,25 | 0,25 |
| Acrylates/C10-30 Alkyl Acrylate Crosspolymer | - | 0,1 |
| Cetearyl Alcohol | 1 | - |
| Lauryl Glucoside (48-55% in Wasser) | 1 | 1 |
| Aqua | ad 100 | ad 100 |
| | | |

| **Befüllung in 200 mL Dose:** | | |
|---|---|---|
| O/W-Emulsion | 94% | 94% |
| Treibmittel | Propan/ Butan/ | Propan/ Butan/ |
| | iso-Butan | iso-Butan |
| Druckstufe | 3,0 bar | 3,0 bar |
| | | |
| Schaumeigenschaften (5 Minuten nach Entnahme) | Fällt nach austragen nicht zusammen / zerläuft nicht | Fällt nach austragen schnell zusammen / zerläuft |

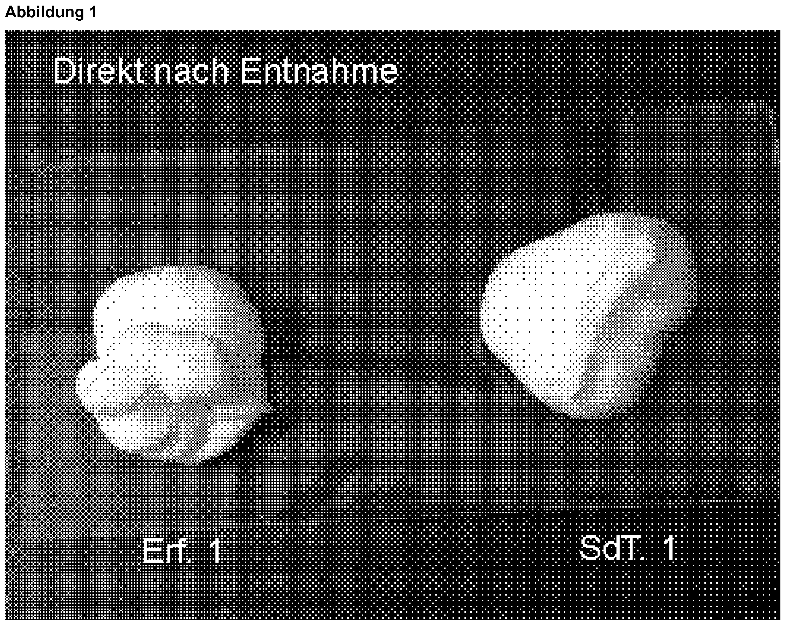

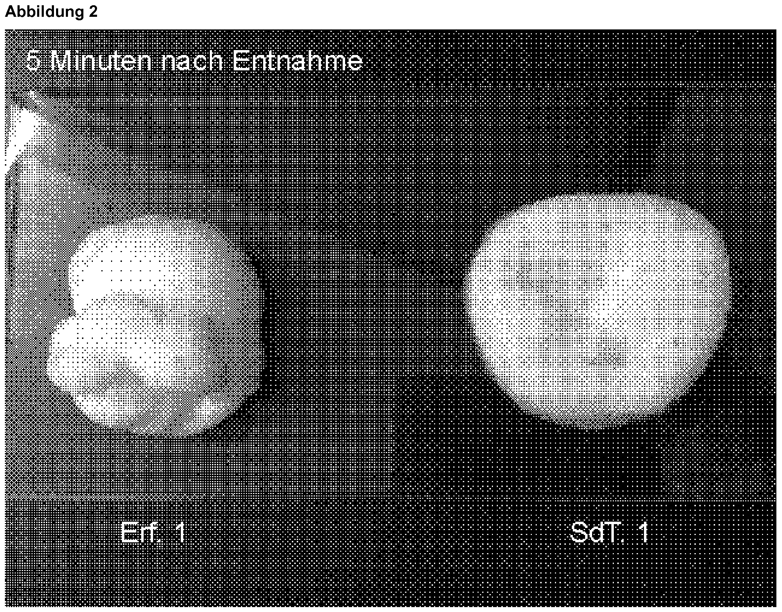

Ein Vergleich des erfindungsgemäß erhaltenen Schaumes mit dem Schaum des nicht erfindungsgemäßen Beispiels (Sdt.1) zeigt, dass 5 Minuten nach Ausbringen der Schaum mit Polyglyceryl-3 Methylglucosestearat deutlich zusammengefallen und zerlaufen ist. Der erfindungsgemäß erhaltene Schaum ist 5 Minuten nach Ausbringen hingegen strukturell stabil, fällt nicht zusammen und verläuft wenig bis gar nicht. Dieses ist den Abbildungen 1 und 2 zu entnehmen. Abbildung 1 zeigt die Schäume direkt nach der Entnahme während die Abbildung 2 die Schäume 5 Minuten nach der Entnahme zeigt.

### Weitere Beispiele:

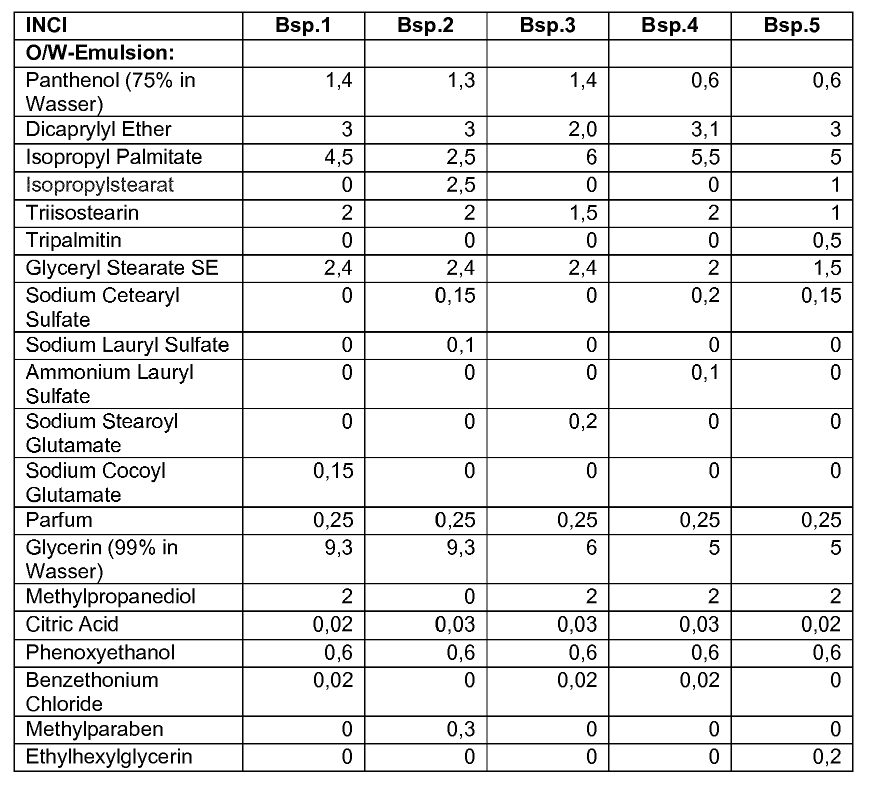

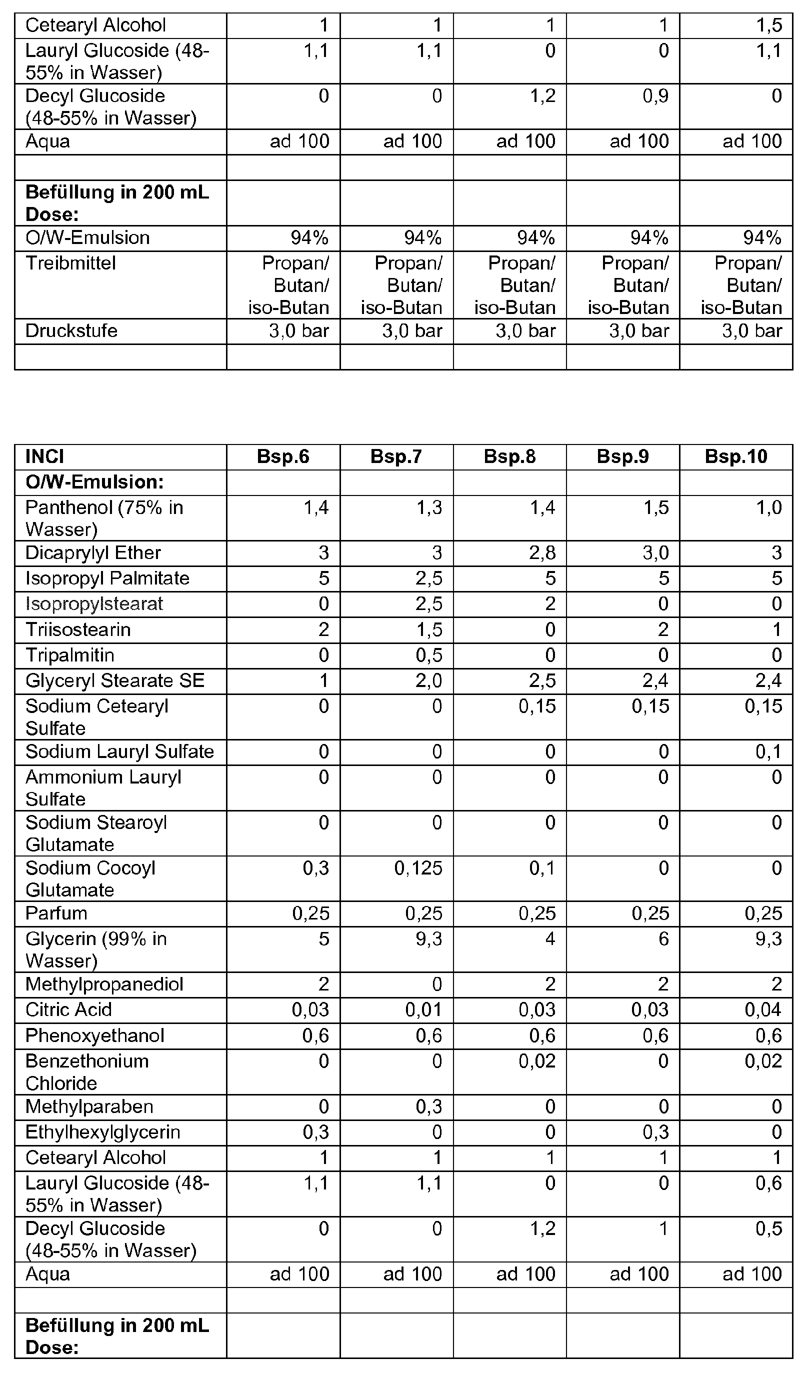

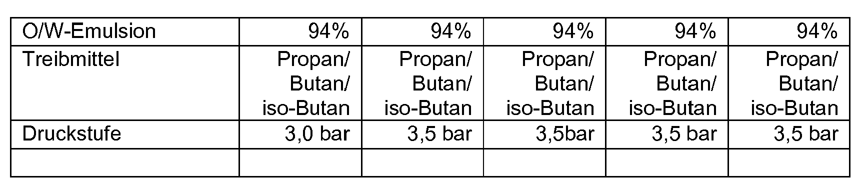

## Patentansprüche

1. Kosmetisches Reinigungsprodukt aufweisend einen Druckbehälter mit Entnahmeventil enthaltend
a) eine Emulsion umfassend
i) mindestens ein Aminosäuretensid gemäß einer der Formeln I bis IV 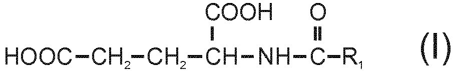 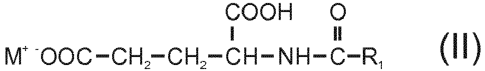 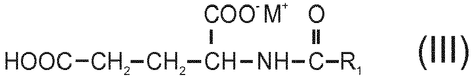 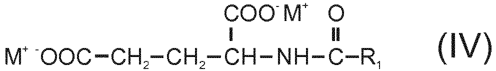 wobei *R₁* eine Alkylkette mit 10 bis 20 Kohlenstoffatomen ist und M⁺ jeweils ein Na⁺ oder NH₄⁺ ist,
und/oder mindestens ein Alkylsulfat gemäß der Formel V 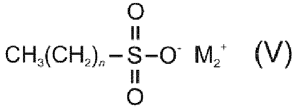 wobei n eine ganzzahlige Zahl im Bereich von 9 bis 19 ist und *M*₂⁺ ein Na⁺ oder NH₄⁺ ist,
ii) Glycerylmonostearat SE, und
iii) mindestens ein Alkylpolyglycosid;
und
b) mindestens ein Treibgas oder Treibgasgemisch.

2. Verwendung einer mit einem Treibgas oder Treibgasgemisch aufgeschäumten Emulsion umfassend
i) mindestens ein Aminosäuretensid gemäß einer der Formeln I bis IV 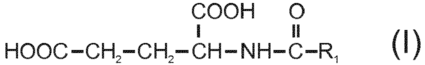 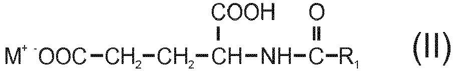 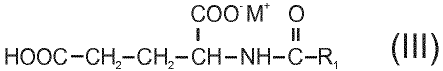 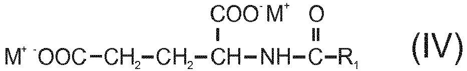 wobei *R₁* eine Alkylkette mit 10 bis 20 Kohlenstoffatomen ist und *M*⁺ jeweils ein Na⁺ oder NH₄⁺ ist,
und/oder mindestens ein Alkylsulfat gemäß der Formel V 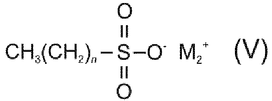 wobei n eine ganzzahlige Zahl im Bereich von 9 bis 19 ist und *M*₂⁺ ein Na⁺ oder NH₄⁺ ist,
ii) Glycerylmonostearat SE, und
iii) mindestens ein Alkylpolyglycosid
zur Bereitstellung eines Schaumes mit einer verbesserten Tragkraft und/oder höheren strukturellen Stabilität.

3. Kosmetisches Produkt oder Verwendung nach einem der vorhergehenden Ansprüche **dadurch gekennzeichnet, dass** die Emulsion mindestens ein Aminosäuretensid gemäß einer der Formeln I bis IV und/oder mindestens ein Alkylsulfat gemäß der Formel V in einem Gesamtanteil 0,05 bis 2 Gew.-%, bevorzugt 0,075 bis 0,5 Gew.-%, bezogen auf das Gesamtgewicht der Emulsion, enthält.

4. Kosmetisches Produkt oder Verwendung nach einem der vorhergehenden Ansprüche **dadurch gekennzeichnet, dass** als Aminosäuretenside Natriumlauroylglutamat, Dinatriumlauroylglutamat, Natriumcocoylglutamat, Dinatriumcocoylglutamat, Natriummyristoylglutamat, Dinatriummyristoylglutamat, Natriumcetoylglutamat, Dinatriumcetoylglutamat, Natriumstearoylglutamat und/oder Dinatriumstearoylglutamat, bevorzugt Natriumstearoylglutamat gewählt werden.

5. Kosmetisches Produkt oder Verwendung nach einem der vorhergehenden Ansprüche **dadurch gekennzeichnet, dass** als Alkylsulfat Natriumlaurylsulfat, Ammoniumlaurylsulfat, Natriummyristylsulfat, Natriumcetylsulfat, Natriumcetearylsulfat und/oder Natriumstearylsulfat, bevorzugt Natriumcetearylsulfat gewählt wird.

6. Kosmetisches Produkt oder Verwendung nach einem der vorhergehenden Ansprüche **dadurch gekennzeichnet, dass** die Emulsion Glycerylmonostearat SE in einem Anteil von 0,1 bis 5 Gew.-%, bevorzugt 1 bis 4 Gew.-% und insbesondere bevorzugt 1,5 bis 3 Gew.-%, bezogen auf das Gesamtgewicht der Emulsion, enthält.

7. Kosmetisches Produkt oder Verwendung nach einem der vorhergehenden Ansprüche **dadurch gekennzeichnet, dass** der Gesamtanteil der Alkylpolyglycoside in der Emulsion von 0,05 Gew-% bis 5 Gew.-%, bevorzugt 0,1 Gew-% bis 2 Gew.-% und insbesondere bevorzugt 0,2 Gew-% bis 1 Gew.-% bezogen auf das Gesamtgewicht der erfindungsgemäßen Emulsion beträgt.

8. Kosmetisches Produkt oder Verwendung nach einem der vorhergehenden Ansprüche **dadurch gekennzeichnet, dass** die in der Emulsion enthaltenen Alkylpolyglycoside eine Struktur gemäß Formel VI 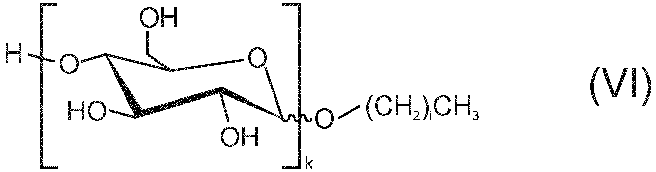 Aufweisen, wobei *k* eine ganzzahlige Zahle von 1 bis 5 ist und in der Alkylkette *i* eine ganzzahle Zahl von 5 bis 20 ist,
und
vorteilhaft Mischungen von Alkylpolyglycoside eingesetzt, die **dadurch gekennzeichnet sind, dass** die in der Emulsion enthaltenen Alkylpolyglycoside die nachfolgenden Anteile an Alkylketten aufweisen:
i=7 in einem Anteil von 24 Gew.-% bis 40 Gew.-%
i=9 in einem Anteil von 15 Gew.-% bis 28 Gew.-%
i=11 in einem Anteil von 27 Gew.-% bis 42 Gew.-%
i=13 in einem Anteil von 9 Gew.-% bis 18 Gew.-%
i=15 in einem Anteil von maximal 4 Gew.-%,
wobei sich die vorstehenden Gew.-% Angaben auf das Gesamtgewicht aller in der Emulsion enthaltenen Alkylpolyglycoside beziehen.

9. Kosmetisches Produkt oder Verwendung nach einem der vorhergehenden Ansprüche **dadurch gekennzeichnet, dass** die Emulsion eine O/W-Emulsion ist.

10. Kosmetisches Produkt oder Verwendung nach einem der vorhergehenden Ansprüche **dadurch gekennzeichnet, dass** ausschließlich Treibgase der Gruppe Propan, n-Butan und Iso-Butan enthalten sind.

11. Kosmetisches Produkt nach einem der vorhergehenden Ansprüche **dadurch gekennzeichnet, dass** im Druckbehälter 90 Gew.-% bis 98 Gew.-% Emulsion und 2 Gew.-% bis 10 Gew.-% Treibgas oder Treibgasgemisch, bevorzugt 92 Gew.-% bis 96 Gew.-% Emulsion und 4 Gew.-% bis 8 Gew.-% Treibgas oder Treibgasgemisch, bezogen auf das Gesamtgewicht aller im Druckbehälter enthaltenen Flüssigkeiten und Gase, enthalten sind.

12. Kosmetisches Produkt oder Verwendung nach einem der vorhergehenden Ansprüche **dadurch gekennzeichnet, dass** die Emulsion ein oder mehr Veresterungsprodukte aus Glycerin mit drei identischen linearen und/oder verzweigten Fettsäuren mit einer Alkylkette mit 12 bis 20 Kohlenstoffatomen in einem Anteil von 0,01 Gew.-% bis 15 Gew.-%, bevorzugt 0,5 Gew.-% bis 10 Gew.-% und insbesondere bevorzugt 1 Gew.-% bis 5 Gew.-%, bezogen auf das Gesamtgewicht der Emulsion, enthält.

13. Kosmetisches Produkt oder Verwendung nach einem der vorhergehenden Ansprüche **dadurch gekennzeichnet, dass** die Emulsion Tripalmitin, Tristearin und Triisostearin enthält, wobei Triisotearin bevorzugt ist.

14. Kosmetisches Produkt oder Verwendung nach einem der vorhergehenden Ansprüche **dadurch gekennzeichnet, dass** die Emulsion keine weiteren Tenside enthält.

15. Kosmetisches Produkt oder Verwendung nach einem der vorhergehenden Ansprüche **dadurch gekennzeichnet, dass** die Emulsion Wachse und Öle in einem Anteil von weniger als 10 Gew.-%, bezogen auf das Gesamtgewicht der Emulsion, enthält.

## Claims

1. Cosmetic cleansing product comprising a pressure container with release valve containing
a) an emulsion comprising
i) at least one amino acid surfactant according to one of the formulae I to IV 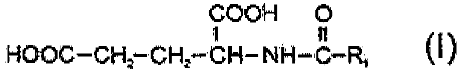 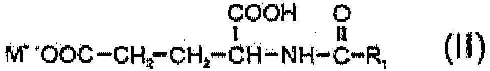 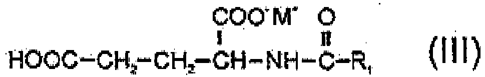 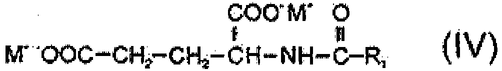 wherein *R₁* is an alkyl chain having 10 to 20 carbon atoms and *M*⁺ is in each case an Na⁺ or NH₄⁺,
and/or at least one alkyl sulfate according to formula V 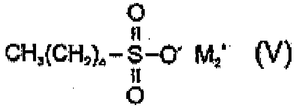 where n is an integer in the range of 9 to 19 and *M*₂⁺ is an Na⁺ or NH₄⁺,
ii) glyceryl monostearate SE, and
iii) at least one alkyl polyglycoside;
and
b) at least one propellant gas or propellant gas mixture.

2. Use of an emulsion foamed with a propellant gas or propellant gas mixture comprising
i) at least one amino acid surfactant according to one of the formulae I to IV 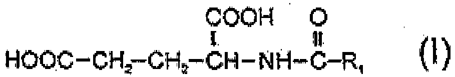 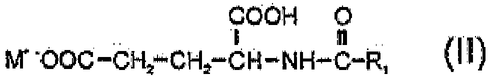 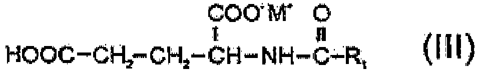 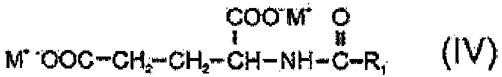 wherein *R₁* is an alkyl chain having 10 to 20 carbon atoms and *M⁺* is in each case an Na⁺ or NH₄⁺,
and/or at least one alkyl sulfate according to formula V 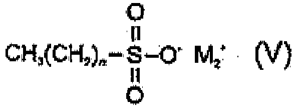 where n is an integer in the range of 9 to 19 and *M₂⁺* is an Na⁺ or NH₄⁺,
ii) glyceryl monostearate SE, and
iii) at least one alkyl polyglycoside for providing a foam having an improved load capacity and/or greater structural stability.

3. Cosmetic product or use according to either of the preceding claims, **characterized in that** the emulsion comprises at least one amino acid surfactant according to one of the formulae I to IV and/or at least one alkyl sulfate according to the formula V in a total proportion of 0.05 to 2% by weight, preferably 0.075 to 0.5% by weight, based on the total weight of the emulsion.

4. Cosmetic product or use according to any of the preceding claims, **characterized in that** the amino acid surfactants selected are sodium lauroyl glutamate, disodium lauroyl glutamate, sodium cocoyl glutamate, disodium cocoyl glutamate, sodium myristoyl glutamate, disodium myristoyl glutamate, sodium cetoyl glutamate, disodium cetoyl glutamate, sodium stearoyl glutamate and/or disodium stearoyl glutamate, preferably sodium stearoyl glutamate.

5. Cosmetic product or use according to any of the preceding claims, **characterized in that** the alkyl sulfate selected is sodium lauryl sulfate, ammonium lauryl sulfate, sodium myristyl sulfate, sodium cetyl sulfate, sodium cetearyl sulfate and/or sodium stearyl sulfate, preferably sodium cetearyl sulfate.

6. Cosmetic product or use according to any of the preceding claims, **characterized in that** the emulsion comprises glyceryl monostearate SE in a proportion from 0.1 to 5% by weight, preferably 1 to 4% by weight and particularly preferably 1.5 to 3% by weight, based on the total weight of the emulsion.

7. Cosmetic product or use according to any of the preceding claims, **characterized in that** the total proportion of alkyl polyglycosides in the emulsion is from 0.05% by weight to 5% by weight, preferably 0.1% by weight to 2% by weight and particularly preferably 0.2% by weight to 1% by weight, based on the total weight of the emulsion according to the invention.

8. Cosmetic product or use according to any of the preceding claims, **characterized in that** the alkyl polyglycosides present in the emulsion have a structure according to formula VI 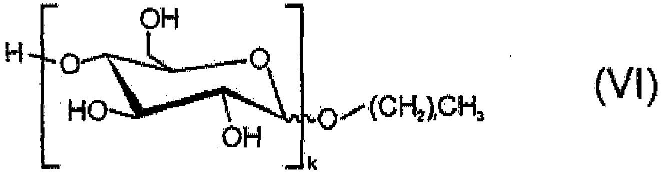 wherein *k* is an integer from 1 to 5, and in the alkyl chain *i* is an integer from 5 to 20,
and
advantageous mixtures of alkyl polyglycosides used are those **characterized in that** the alkyl polyglycosides present in the emulsion have the following proportions of alkyl chains:
i = 7 in a proportion of 24% by weight to 40% by weight
i = 9 in a proportion of 15% by weight to 28% by weight
i = 11 in a proportion of 27% by weight to 42% by weight
i = 13 in a proportion of 9% by weight to 18% by weight
i = 15 in a proportion of at most 4% by weight
wherein the % by weight figures above refer to the total weight of all alkyl polyglycosides present in the emulsion.

9. Cosmetic product or use according to any of the preceding claims, **characterized in that** the emulsion is an O/W emulsion.

10. Cosmetic product or use according to any of the preceding claims, **characterised in that** propellant gases from the group comprising propane, n-butane and isobutane are exclusively present.

11. Cosmetic product according to any of the preceding claims, **characterized in that** 90% by weight to 98% by weight emulsion and 2% by weight to 10% by weight propellant gas or propellant gas mixture, preferably 92% by weight to 96% by weight emulsion and 4% by weight to 8% by weight propellant gas or propellant gas mixture, are present in the pressure container, based on the total weight of all liquids and gases present in the pressure container.

12. Cosmetic product or use according to any of the preceding claims, **characterised in that** the emulsion comprises one or more esterification products of glycerol with three identical linear and/or branched fatty acids with an alkyl chain having 12 to 20 carbon atoms, in a proportion of 0.01% by weight to 15% by weight, preferably 0.5% by weight to 10% by weight and particularly preferably 1% by weight to 5% by weight, based on the total weight of the emulsion.

13. Cosmetic product or use according to any of the preceding claims, **characterized in that** the emulsion comprises tripalmitin, tristearin and triisostearin, preference being given to triisostearin,

14. Cosmetic product or use according to any of the preceding claims, **characterized in that** the emulsion does not comprise any further surfactants.

15. Cosmetic product or use according to any of the preceding claims, **characterized in that** the emulsion comprises waxes and oils in a proportion of less than 10% by weight, based on the total weight of the emulsion.

## Revendications

1. Produit nettoyant cosmétique comprenant un contenant sous pression muni d'une vanne de prélèvement, contenant :
a) une émulsion comprenant :
i) au moins un tensioactif acide aminé selon l'une quelconque des formules I à IV : 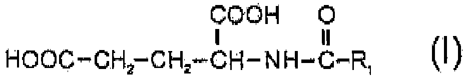 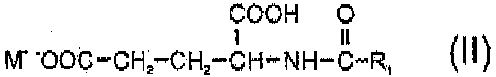 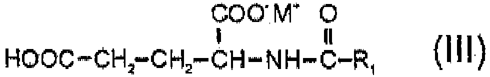 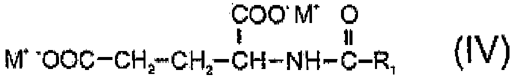 dans lesquelles *R₁* représente une chaîne alkyle de 10 à 20 atomes de carbone, et *M*⁺ représente à chaque fois Na⁺ ou NH₄⁺,
et/ou au moins un sulfate d'alkyle selon la formule V : 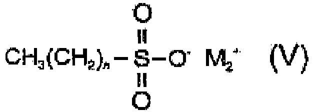 dans laquelle n représente un nombre entier dans la plage allant de 9 à 19, et *M₂*⁺ représente Na⁺ ou NH₄⁺.
ii) du monostéarate de glycéryle SE, et
iii) au moins un polyglycoside d'alkyle ;
et
b) au moins un gaz propulseur ou un mélange de gaz propulseurs.

2. Utilisation d'une émulsion moussée avec un gaz propulseur ou un mélange de gaz propulseurs, comprenant :
i) au moins un tensioactif acide aminé selon l'une quelconque des formules I à IV : 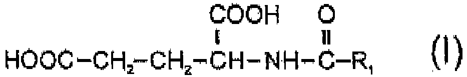 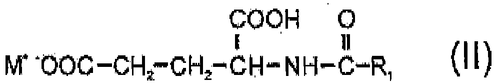 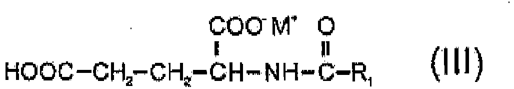 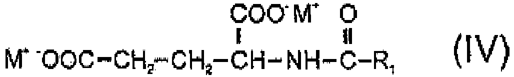 dans lesquelles *R₁* représente une chaîne alkyle de 10 à 20 atomes de carbone, et *M*⁺ représente à chaque fois Na⁺ ou NH₄⁺.
et/ou au moins un sulfate d'alkyle selon la formule V : 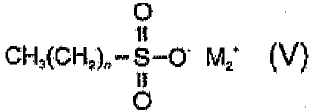 dans laquelle n représente un nombre entier dans la plage allant de 9 à 19, et *M₂*⁺ représente Na⁺ ou NH₄⁺,
ii) du monostéarate de glycéryle SE, et
iii) au moins un polyglycoside d'alkyle,
pour la préparation d'une mousse ayant une capacité de charge améliorée et/ou une stabilité structurale plus élevée.

3. Produit cosmétique ou utilisation selon l'une quelconque des revendications précédentes, caractérisé ou **caractérisée en ce que** l'émulsion contient au moins un tensioactif acide aminé selon l'une quelconque des formules I à IV et/ou au moins un sulfate d'alkyle selon la formule V en une proportion totale de 0,05 à 2 % en poids, de préférence de 0,075 à 0,5 % en poids, par rapport au poids total de l'émulsion.

4. Produit cosmétique ou utilisation selon l'une quelconque des revendications précédentes, caractérisé ou **caractérisée en ce que** le glutamate de lauroyle de sodium, le glutamate de lauroyle de disodium, le glutamate de cocoyle de sodium, le glutamate de cocoyle de disodium, le glutamate de myristoyle de sodium, le glutamate de myristoyle de disodium, le glutamate de cétoyle de sodium, le glutamate de cétoyle de disodium, le glutamate de stéaroyle de sodium et/ou le glutamate de stéaroyle de disodium, de préférence le glutamate de stéaroyle de sodium, sont choisis en tant que tensioactifs acides aminés.

5. Produit cosmétique ou utilisation selon l'une quelconque des revendications précédentes, caractérisé ou **caractérisée en ce que** le sulfate de lauryle de sodium, le sulfate de lauryle d'ammonium, le sulfate de myristyle de sodium, le sulfate de cétyle de sodium, le sulfate de cétéaryle de sodium et/ou le sulfate de stéaryle de sodium, de préférence le sulfate de cétéaryle de sodium, est choisi en tant que sulfate d'alkyle.

6. Produit cosmétique ou utilisation selon l'une quelconque des revendications précédentes, caractérisé ou **caractérisée en ce que** l'émulsion contient le monostéarate de glycéryle SE en une proportion de 0,1 à 5 % en poids, de préférence de 1 à 4 % en poids, et de manière particulièrement préférée de 1,5 à 3 % en poids, par rapport au poids total de l'émulsion.

7. Produit cosmétique ou utilisation selon l'une quelconque des revendications précédentes, caractérisé ou **caractérisée en ce que** la proportion totale des polyglycosides d'alkyle dans l'émulsion est de 0,05 % en poids à 5 % en poids, de préférence de 0,1 % en poids à 2 % en poids, et de manière particulièrement préférée de 0,2 % en poids à 1 % en poids, par rapport au poids total de l'émulsion selon l'invention.

8. Produit cosmétique ou utilisation selon l'une quelconque des revendications précédentes, caractérisé ou **caractérisée en ce que** les polyglycosides d'alkyle contenus dans l'émulsion présentent une structure selon la formule VI : 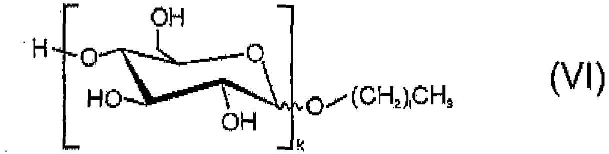 dans laquelle *k* représente un nombre entier de 1 à 5, et *i* dans la chaîne alkyle représente un nombre entier de 5 à 20,
et
des mélanges de polyglycosides d'alkyle sont avantageusement utilisés, qui sont **caractérisés en ce que** les polyglycosides d'alkyle contenus dans l'émulsion présentent les proportions suivantes de chaînes alkyle :
i = 7 en une proportion de 24 % en poids à 40 % en poids,
i = 9 en une proportion de 15 % en poids à 28 % en poids,
i = 11 en une proportion, de 27 % en poids à 42 % en poids,
i = 13 en une proportion de 9 % en poids à 18 % en poids,
i = 15 en une proportion d'au plus 4 % en poids,
les données de % en poids précédentes se rapportant au poids total de tous les polyglycosides d'alkyle contenus dans l'émulsion.

9. Produit cosmétique ou utilisation selon l'une quelconque des revendications précédentes, caractérisé ou **caractérisée en ce que** l'émulsion est une émulsion H/E.

10. Produit cosmétique ou utilisation selon l'une quelconque des revendications précédentes, caractérisé ou **caractérisée en ce qu'**exclusivement des gaz propulseurs du groupe constitué par le propane, le n-butane et l'iso-butane sont contenus.

11. Produit cosmétique selon l'une quelconque des revendications précédentes, **caractérisé en ce que** 90 % en poids à 98 % en poids d'émulsion et 2 % en poids à 10 % en poids de gaz propulseur ou de mélange de gaz propulseurs, de préférence 92 % en poids à 96 % en poids d'émulsion et 4 % en poids à 8 % en poids de gaz propulseur ou de mélange de gaz propulseurs, sont contenus dans le contenant sous pression, par rapport au poids total de tous les liquides et les gaz contenus dans le contenant sous pression.

12. Produit cosmétique ou utilisation selon l'une quelconque des revendications précédentes, caractérisé ou **caractérisée en ce que** l'émulsion contient un ou plusieurs produits d'estérification composés de glycérine avec trois acides gras linéaires et/ou ramifiés identiques contenant une chaîne alkyle de 12 à 20 atomes de carbone en une proportion de 0,01 % en poids à 15 % en poids, de préférence de 0,5 % en poids à 10 % en poids, et de manière particulièrement préférée de 1 % en poids à 5 % en poids, par rapport au poids total de l'émulsion.

13. Produit cosmétique ou utilisation selon l'une quelconque des revendications précédentes, caractérisé ou, **caractérisée en ce que** l'émulsion contient de la tripalmitine, de la tristéarine et de la triisostéarine, la triisostéarine étant préférée.

14. Produit cosmétique ou utilisation selon l'une quelconque des revendications précédentes, caractérisé ou **caractérisée en ce que** l'émulsion ne contient pas de tensioactifs supplémentaires.

15. Produit cosmétique ou utilisation selon l'une quelconque des revendications précédentes, caractérisé ou **caractérisée en ce que** l'émulsion contient des cires et des huiles en une proportion de moins de 10 % en poids, par rapport au poids total de l'émulsion.
